(19) Europäisches Patentamt
European Patent Office
Office européen des brevets

(11) Veröffentlichungsnummer: **0 070 370**
**A1**

(12) # EUROPÄISCHE PATENTANMELDUNG

(21) Anmeldenummer: **82104389.0**

(22) Anmeldetag: **19.05.82**

(51) Int. Cl.[3]: **C 07 D 309/06**, C 07 D 309/22, C 07 D 307/14, C 07 D 307/16, C 07 D 317/28, C 07 D 317/30, C 07 D 319/06, C 07 D 335/02, C 07 D 339/06, A 01 N 43/02

(30) Priorität: **29.05.81 DE 3121355**

(43) Veröffentlichungstag der Anmeldung: **26.01.83**
**Patentblatt 83/4**

(84) Benannte Vertragsstaaten: **AT BE CH DE FR GB IT LI LU NL SE**

(71) Anmelder: **BASF Aktiengesellschaft,**
**Carl-Bosch-Strasse 38, D-6700 Ludwigshafen (DE)**

(72) Erfinder: **Becker, Rainer, Dr., Sonnenwendstrasse 83, D-6702 Bad Duerkheim (DE)**
Erfinder: **Jahn, Dieter, Dr., Burgunder Weg 8, D-6803 Neckarhausen (DE)**
Erfinder: **Rohr, Wolfgang, Dr., In der Dreispitz 13, D-6706 Wachenheim (DE)**
Erfinder: **Himmele, Walter, Dr., Eichenweg 14, D-6909 Walldorf (DE)**
Erfinder: **Siegel, Hardo, Dr., Hans-Purrmann-Allee 25, D-6720 Speyer (DE)**
Erfinder: **Wuerzer, Bruno, Dr., Ruedigerstrasse 13, D-6701 Otterstadt (DE)**

(54) **Cyclohexandionderivate, Verfahren zu ihrer Herstellung und diese enthaltende Herbizide.**

(57) Die vorliegende Anmeldung betrifft Cyclohexandionderivate der allgemeinen Formel

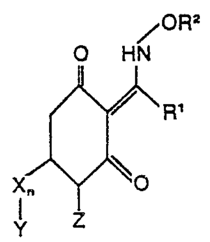

in der
$R^1$ Alkyl
$R^2$ Alkyl, Alkenyl, Alkinyl, Halogenalkenyl
X Alkylenrest
n =0 oder 1
Y Heterocyclus
Z Wasserstoff oder Methoxycarbonyl bedeutet,
sowie die Salze dieser Verbindung und diese enthaltende Herbizide.

EP 0 070 370 A1

# Cyclohexandionderivate, Verfahren zu ihrer Herstellung und diese enthaltende Herbizide

Die vorliegende Erfindung betrifft neue Cyclohexan-1,3-dionderivate, Verfahren zur Herstellung dieser Verbindungen sowie Herbizide, welche diese Verbindungen enthalten.

Cyclohexandionderivate mit Thienyl- oder Furylsubstitution in 5-Position mit relativ geringer herbizider Wirkung sind bekannt (DE-AS 24 39 104).

Es wurde nun gefunden, daß Verbindungen der allgemeinen Formel I

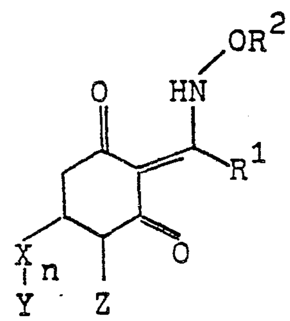

I

in der

$R^1$ Alkyl mit 1 - 4 Kohlenstoffatomen

$R^2$ Alkyl mit 1 - 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 - 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 - 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet

sowie die Salze dieser Verbindungen unerwünschte Pflanzen aus der Familie der Gräser sehr gut bekämpfen und gleichzeitig als selektive Herbizide ein hohes Maß an Verträglichkeit für breitblättrige und andere nicht zu der Familie der Gräser zählende Kulturpflanzen besitzen.

$R^1$ bedeutet beispielsweise Propyl, Ethyl, Butyl,
$R^2$ bedeutet beispielsweise Methyl, Ethyl, Propyl, Allyl, 2-Chlorallyl, 3-Chlorallyl,
X bedeutet beispielsweise Methylen, Ethylen,
Y bedeutet beispielsweise Tetrahydropyranyl, Dihydropyranyl, Methyltetrahydropyranyl, Dioxanyl, Dioxolanyl, Dithiolanyl, Dihydrothiopyranyl.

Die neuen Verbindungen können in verschiedenen tautomeren Formen vorliegen:

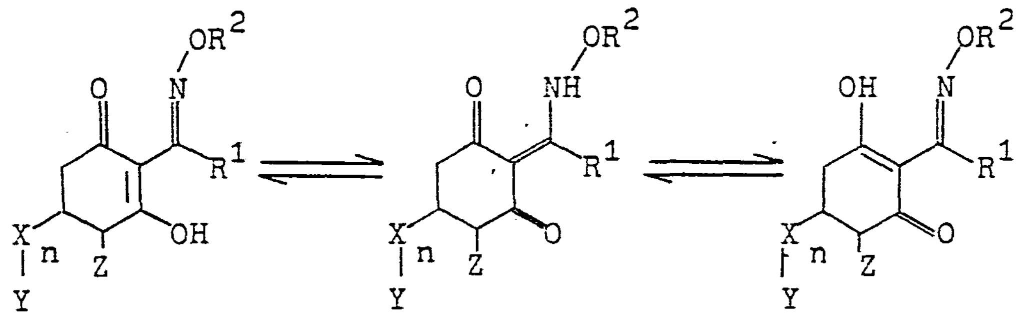

Die vorliegende Erfindung umfaßt alle diese Formen.

Zur Herstellung der neuen Verbindungen ist beispielsweise der nachfolgend beschriebene Weg geeignet:

a) 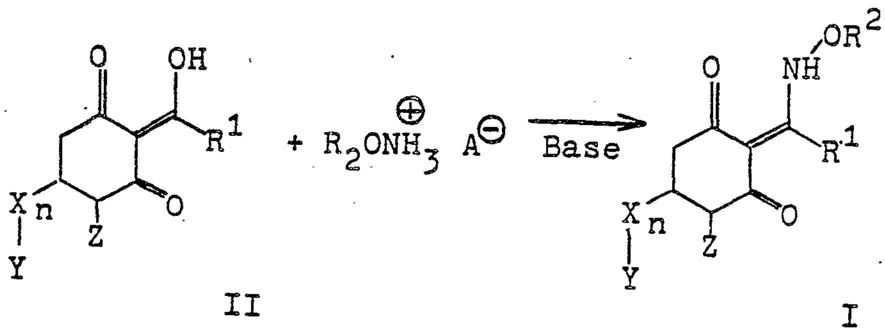

II I

wobei $R^1$, $R^2$, X, Y, Z, A die oben genannte Bedeutung haben.

Man führt die Reaktion zweckmäßig in heterogener Phase in einem inerten Lösungsmittel bei Temperaturen zwischen 0 und 80°C in Gegenwart einer Base durch. Basen sind beispielsweise Carbonate, Hydrogencarbonate, Acetate, Alkoholate, Hydroxide oder Oxide von Alkali- oder Erdalkalimetallen, besonders von Natrium und Kalium sowie Magnesium und Kalium. Daneben können auch organische Basen wie Pyridin oder tertiäre Amine Verwendung finden.

Ein für die Umsetzung besonders geeigneter definierter pH-Bereich reicht von pH 2 bis pH 7, insbesondere von pH 4,5 bis pH 5,5. Die Einstellung des pH-Bereichs für die Umsetzung erfolgt vorteilhaft durch Zusatz von Acetaten, beispielsweise Alkaliacetaten, insbesondere Natrium- oder Kaliumacetat oder ihren Mischungen. Die Alkaliacetate werden beispielsweise angewendet in Mengen von 0,5 bis 2 mol, bezogen auf die Ammoniumverbindung.

Als Lösungsmittel sind geeignet beispielsweise Methanol, Ethanol, Isopropanol, Benzol, Tetrahydrofuran, Chloroform, Acetonitril, Dichlorethan, Essigsäureethylester, Dioxan, Dimethylsulfoxid.

Die Reaktion ist nach einigen Stunden beendet, das Reaktionsprodukt kann durch Einengen der Mischung, Zugabe von Wasser und Extraktion mit einem unpolaren Lösungsmittel sowie Abdestillieren des Lösungsmittels unter vermindertem Druck, isoliert werden.

b) Darüber hinaus ist die Herstellung der neuen Verbindungen auch durch Umsetzung der Verbindungen II mit den entsprechenden Aminen $R^2$-$ONH_2$ durchführbar.

c) Weiterhin ist die Herstellung der neuen Derivate auch durch Alkylierung der Oxime mit Alkylierungsmitteln möglich:

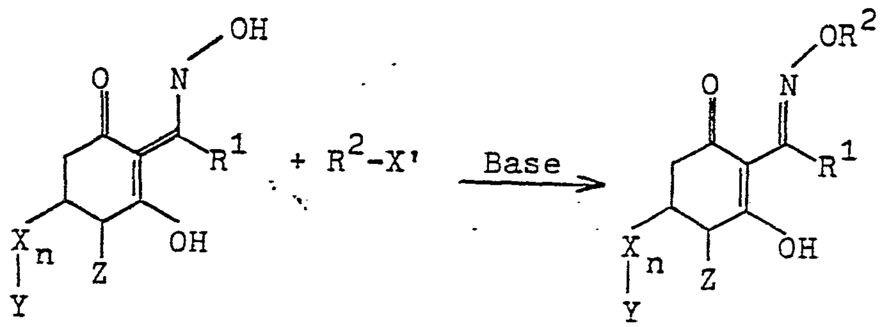


Das Verfahren a) wird bevorzugt.

Die Verbindungen der Formel II können durch Acylierung der Cyclohexan-1,3-dione III, wie dies in Tetrahedron Letters 29, 2491 beschrieben ist, erhalten werden. Die Verbindungen III können ebenfalls in tautomeren Formen vorliegen.

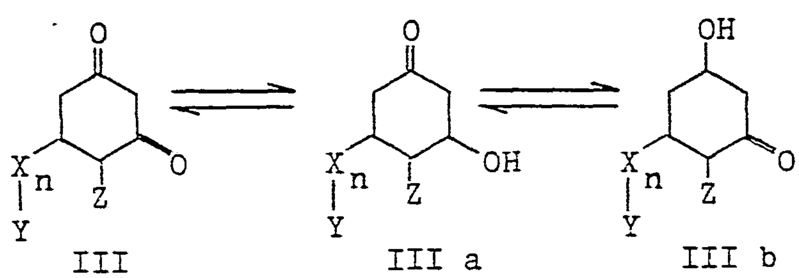

III III a III b

Verbindungen der Formel III sind aus Aldehyden $Y$-$X_n$-$CH$=$O$ nach literaturbekannten Methoden beispielsweise durch Aldolkondensation mit Keton und anschließender Cyclisierung mit Malonsäureestern analog Organic Synthesis Coll. Vol. II, Seite 200 herstellbar. Auch durch Umsetzung des Aldehyds $Y-X_n-CH=O$ mit Malonsäure nach Knoevenagel-Döbner (s. Org. Reaktions Bd. 15, Seite 204), Veresterung der erhaltenen Säure sowie Cyclisierung mit Acetessigester, in analoger Weise wie dies z.B. in Chem. Ber. 96, Seite 2946 beschrieben wird, gelangt man zu den Zwischenprodukten der Formel III.

Die Salze der Verbindungen sind beispielsweise die Alkalisalze, insbesondere Natrium- oder Kaliumsalze.

Die Natrium- und Kaliumsalze der neuen Verbindungen können durch Behandeln dieser Verbindungen mit Natrium- oder Kaliumhydroxid in wäßriger Lösung oder in einem organischen Lösungsmittel wie Methanol, Ethanol, Aceton erhalten werden. Es können auch Alkalialkoholate als Basen eingesetzt werden.

Andere Metallsalze, z.B. die Mangan-, Kupfer-, Zink-, Eisen- oder Bariumsalze können aus dem Natriumsalz durch Reaktion mit dem entsprechenden Metallchlorid in wäßriger Lösung hergestellt werden. Die folgenden Beispiele erläutern die Herstellung der neuen Cyclohexandione (Gewichtsteile verhalten sich zu Volumenteilen wie Kilogramm zu Liter).

Beispiel 1

10,0 Gewichtsteile 2-Butyryl-4-methoxycarbonyl-5[-tetrahydropyran-4-ylmethyl]-cyclohexan-1,3-dion wurden in 150 Volumenteilen Ethanol gelöst und mit 2,93 Gewichtsteilen Ethyloxiammoniumchlorid sowie 2,71 Gewichtsteilen wasserfreiem Natriumacetat versetzt. Nach 20stündigem Rühren bei

20°C wurde in Eiswasser gegeben und mit Methylenchlorid extrahiert. Nach dem Einengen der organischen Phase verblieben 10,5 Gewichtsteile 2(1-Ethoxyaminobutyliden)-4-methoxycarbonyl-5-[tetrahydropyran-4-ylmethyl-]cyclohexan-1,3-dion (Verbindung Nr. 1) als zähes Öl mit folgender Struktur:

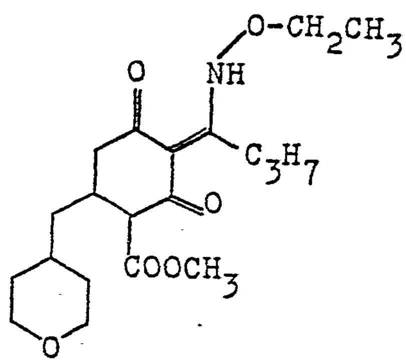

$C_{20}H_{31}O_6N$ M = 381

| | | | | | | |
|---|---|---|---|---|---|---|
| Ber | C | 63,0 | H | 8,2 | N | 3,7 |
| Gef | C | 63,3 | H | 8,1 | N | 3,7 |

Beispiel 2

10,0 Gewichtsteile 2-Butyryl-5[2-(1,3-dioxan-2-yl-)ethyl]-cyclohexan-1,3-dion wurden in 150 Volumenteilen Ethanol gelöst und mit 3,72 Gewichtsteilen Allyloxiammoniumchlorid sowie 3,03 Gewichtsteilen wasserfreiem Natriumacetat versetzt und 20 Stunden bei 20°C gerührt. Anschließend wurde die Suspension in Eiswasser eingerührt und mit Methylenchlorid extrahiert. Nach Einengen der organischen Phase verblieben 11,5 Gewichtsteile 2-(1-Allyloxiaminobutyliden)-5-[2-(1,3-dioxan-2-yl-)ethyl]-cyclohexan-1,3-dion (Verbindung Nr. 2) als Feststoff mit folgender Struktur (Schmelzpunkt 50 bis 52°C):

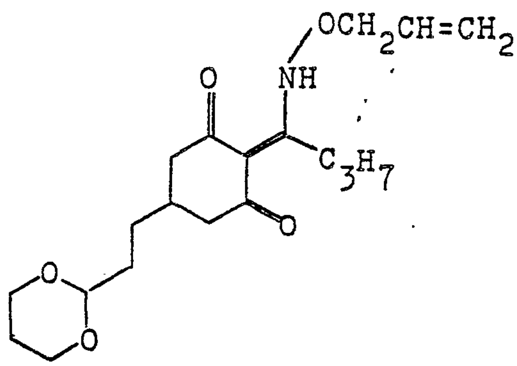

$C_{19}H_{29}O_5N$ M = 351

| | | | | | | |
|---|---|---|---|---|---|---|
| Ber: | C | 64,9 | H | 8,3 | N | 4,0 |
| Gef: | C | 65,1 | H | 8,1 | N | 3,7 |

Beispiel 3

12,0 Gewichtsteile 2-Butyryl-4-methoxycarbonyl-5-[2-(1,3-dithiolan-2-yl-)-ethyl]-cyclohexan-1,3-dion wurden in 150 Volumenteilen Ethanol gelöst und mit 3,29 Gewichtsteilen Allyloxiammoniumchlorid sowie 3,28 g wasserfreiem Natriumacetat versetzt. Nach 20stündigem Rühren bei 20°C wurde auf Eiswasser gegeben und mit Methylenchlorid extrahiert. Nach Einengen der organischen Phase verblieben 13,1 Gewichtsteile 2-(1-Allyloxiaminobutyliden)-4-methoxycarbonyl-5-[2-(1,3-dithiolan-2-yl-)-ethyl]-cyclohexan-1,3-dion (Verbindung Nr. 3) als zähes Öl mit nachstehender Struktur:

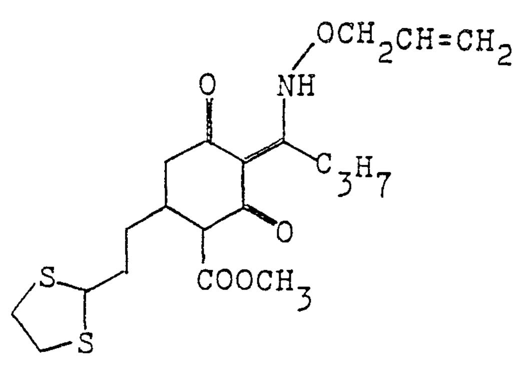

$C_{20}H_{29}O_5NS_2$ M = 427

| | | | | | | | | |
|---|---|---|---|---|---|---|---|---|
| Ber: | C | 56,2 | H | 6,8 | N | 2,3 | S | 15,0 |
| Gef: | C | 57,0 | H | 6,7 | N | 2,8 | S | 14,7 |

Die folgenden Verbindungen wurden in entsprechender Weise erhalten:

| Verbindung Nr. | $R^1$ | $R^2$ | $X_n-Y$ | Z | Fp oder Brechungsindex |
|---|---|---|---|---|---|
| 4 | Propyl | Allyl | Tetrahydropyran-4-ylmethyl | $COOCH_3$ | |
| 5 | " | Ethyl | " | H | |
| 6 | " | Allyl | " | H | |
| 7 | " | Allyl | 2-(1,3-Dioxan-2-yl-)ethyl | $COOCH_3$ | |
| 8 | " | Ethyl | " | H | |
| 9 | " | Allyl | 4-Methyltetrahydropyran-3-yl | $COOCH_3$ | |
| 10 | " | Ethyl | " | H | $n_D^{22}$ 1,5235 |
| 11 | " | Allyl | " | H | $n_D^{22}$ 1,5297 |
| 12 | " | Ethyl | 1-(4-Methyl-1,3-dioxan-2-yl-) 2-methyl-propyl | $COOCH_3$ | |
| 13 | " | Allyl | " | $COOCH_3$ | |
| 14 | " | Ethyl | " | H | |
| 15 | " | Ethyl | 1-Phenyl-2-(1,3-dioxolan-2-yl-)ethyl | $COOCH_3$ | |
| 16 | " | Allyl | " | $COOCH_3$ | |
| 17 | " | Ethyl | (2-H)-5,6-Dihydropyran-3-yl | $COOCH_3$ | |
| 18 | " | Allyl | " | $COOCH_3$ | |
| 19 | " | Ethyl | " | H | $n_D^{20}$ 1,5339 |

| Verbindung Nr. | $R^1$ | $R^2$ | $X_n-Y$ | Z | Fp oder Brechungsindex |
|---|---|---|---|---|---|
| 20 | Propyl | Ethyl | (4-H)-2,3-Dihydropyran-2-yl | $COOCH_3$ | $n_D^{26}$ 1,5225 |
| 21 | " | Allyl | (4-H)-2,3-Dihydropyran-3-yl | $COOCH_3$ | $n_D^{27}$ 1,5262 |
| 22 | " | Ethyl | Tetrahydropyran-2-yl | $COOCH_3$ | $n_D^{24}$ 1,5142 |
| 23 | " | Allyl | " | $COOCH_3$ | $n_D^{25}$ 1,5204 |
| 24 | " | Ethyl | " | H | $n_D^{26}$ 1,5136 |
| 25 | " | Allyl | " | H | $n_D^{27}$ 1,5200 |
| 26 | " | Allyl | " | H | $n_D^{24}$ 1,5149 |
| 27 | " | 3-Chlorallyl | Tetrahydropyran-4-ylmethyl | $COOCH_3$ | Fp 75-79° |
| 28 | " | 2-Chlorallyl | " | $COOCH_3$ | Fp 72-75° |
| 31 | " | 3-Chlorallyl | " | H | |
| 32 | " | 2-Chlorallyl | " | H | |
| 36 | " | " | 2-(1,3-Dioxan-2-yl-)ethyl | H | Fp 55-58° |

| Verbindung Nr. | $R^1$ | $R^2$ | $X_n-Y$ | Z | Fp oder Brechungsindex |
|---|---|---|---|---|---|
| 37 | Propyl | 3-Chlorallyl | 2-(1,3-Dioxan-2-yl-)ethyl | H | $n_D^{22}$ 1,5281 |
| 38 | " | 2,3,3-Trichlorallyl | " | H | $n_D^{22}$ 1,5401 |
| 41 | " | 3-Chlorallyl | 4-Methyltetrahydropyran-3-yl | H | $n_D^{22}$ 1,5389 |
| 44 | " | Allyl | 1-Phenyl-2-(1,3-dioxolan-2-yl-)ethyl | H | |
| 48 | " | Ethyl | (4-H)-2,5-Dimethyl-2,3-dihydropyran-2-yl | H | $n_D^{18}$ 1,5259 |
| 49 | " | Allyl | " | H | $n_D^{18}$ 1,5301 |
| 61 | " | Ethyl | (2-H)-5,6-Dihydrothiopyran-3-yl | H | $n_D^{23}$ 1,5620 |
| 62 | " | Allyl | " | H | $n_D^{23}$ 1,5678 |
| 65 | " | Ethyl | (2-H)-2,6-Dimethyl-5,6-di- | H | $n_D^{23}$ 1,5464 |
| 66 | " | Allyl | " | H | $n_D^{23}$ 1,5510 |
| 68 | " | 2,3,3-Trichlorallyl | 4-Methyltetrahydropyran-3-yl | H | |

| Verbindung Nr. | $R^1$ | $R^2$ | $X_n-Y$ | Z | Fp oder Brechungsindex |
|---|---|---|---|---|---|
| 71 | Propyl | Propargyl | 4-Methyltetrahydropyran-3-yl | H | $n_D^{23}$ 1,5332 |
| 72 | " | Propyl | " | H | $n_D^{26}$ 1,5181 |
| 76 | " | Allyl | (2-H)-5,6-Dihydropyran-3-yl | H | $n_D^{18}$ 1,5449 |
| 77 | Ethyl | Ethyl | Tetrahydropyran-2-yl | H | $n_D^{31}$ 1,5199 |
| 78 | " | Allyl | " | H | $n_D^{31}$ 1,5265 |
| 79 | Propyl | Allyl | Tetrahydropyran-3-yl | H | $n_D^{18}$ 1,5313 |
| 80 | Ethyl | Ethyl | " | H | Fp 38-40° |
| 81 | Ethyl | Allyl | " | H | $n_D^{18}$ 1,5342 |
| 83 | " | Ethyl | (2-H)-2,6-Dimethyl-5,6-dihydrothiopyran-3-yl | H | $n_D^{23}$ 1,5549 |
| 84 | " | Allyl | " | H | $n_D^{23}$ 1,5608 |

| Verbindung Nr. | $R^1$ | $R^2$ | $X_n$-Y | Z | Fp oder Brechungsindex |
|---|---|---|---|---|---|
| 85 | Ethyl | Ethyl | (2-H)-5,6-Dihydrothiopyran-3-yl | H | $n_D^{23}$ 1,5689 |
| 86 | " | Allyl | " | H | $n_D^{23}$ 1,5727 |
| 91 | Propyl | Ethyl | Tetrahydrofuran-2-yl | H | $n_D^{21}$ 1,5179 |
| 92 | " | Allyl | " | H | $n_D^{21}$ 1,5261 |
| 99 | " | Ethyl | (2-H)-2,6-Dimethyl-5,6-dihydropyran-3-yl | H | $n_D^{29}$ 1,5149 |
| 100 | " | Allyl | " | H | $n_D^{29}$ 1,5275 |
| 112 | Natriumsalz der Verbindung Nr. 26 | | | | Fp 168-172° (Zers.) |

Die folgenden Verbindungen können in entsprechender Weise erhalten werden:

| Verbindung Nr. | $R^1$ | $R^2$ | $X_n-Y$ | Z |
|---|---|---|---|---|
| 29 | Propyl | 2,3,3-Trichlorallyl | Tetrahydropyran-4-ylmethyl | $COOCH_3$ |
| 30 | " | 2,3-Dibromallyl | " | $COOCH_3$ |
| 33 | " | " | " | H |
| 34 | " | 3-Chlorallyl | 2-(1,3-Dioxan-2-yl-)ethyl | $COOCH_3$ |
| 35 | " | 2-Chlorallyl | " | $COOCH_3$ |
| 39 | " | 2,3-Dichlorallyl | " | H |
| 40 | " | 2,3-Dibromallyl | " | H |
| 42 | " | 3-Chlorallyl | 1-(4-Methyl-1,3-dioxan-2-yl-)-2-methyl-propyl | H |
| 43 | " | " | 1-Phenyl-2-(1,3-dioxolan-2-yl-)ethyl | H |
| 45 | " | Allyl | 2-(1,3-Dithiolan-2-yl-)ethyl | H |
| 46 | " | Ethyl | " | H |
| 47 | " | 3-Chlorallyl | " | H |
| 50 | " | " | (4-H)-2,5-Dimethyl-2,3-dihydropyran-2-yl | H |
| 51 | " | Ethyl | 2,5-Dimethyltetrahydropyran-2-yl | H |
| 52 | " | 3-Chlorallyl | " | H |

| Verbindung Nr. | $R^1$ | $R^2$ | $X_n-Y$ | Z |
|---|---|---|---|---|
| 53 | Propyl | 3-Chlorallyl | (2-H)-5,6-Dihydropyran-3-yl | H |
| 54 | " | Ethyl | (4-H)-2,3-Dihydropyran-2-yl | H |
| 55 | " | Allyl | " | H |
| 56 | " | 3-Chlorallyl | " | H |
| 57 | " | " | Tetrahydropyran-3-yl | H |
| 58 | " | " | Tetrahydropyran-2-yl | H |
| 59 | " | " | (2-H)-2,6-Dimethyl-5,6-dihydrothiopyran-3-yl | H |
| 60 | " | Ethyl | (2-H)-5,6-Dihydrothiopyran-3-yl | $COOCH_3$ |
| 63 | " | 3-Chlorallyl | " | H |
| 64 | " | Allyl | (2-H)-2,6-Dimethyl-5,6-dihydrothiopyran-3-yl | $COOCH_3$ |
| 67 | " | 2-Chlorallyl | 4-Methyltetrahydropyran-3-yl | H |
| 69 | Ethyl | Ethyl | " | H |
| 70 | " | Allyl | " | H |
| 73 | Propyl | Butyl | " | H |
| 74 | Ethyl | Ethyl | (2-H)-5,6-Dihydropyran-3-yl | H |
| 75 | " | Allyl | " | H |

| Verbindung Nr. | $R^1$ | $R^2$ | $X_n-Y$ | Z |
|---|---|---|---|---|
| 82 | Propyl | Allyl | 2,5-Dimethyltetrahydropyran-2-yl | H |
| 87 | " | Ethyl | Tetrahydrothiopyran-3-yl | H |
| 88 | " | Allyl | " | H |
| 89 | Ethyl | Ethyl | " | H |
| 90 | " | Allyl | " | H |
| 93 | Propyl | Ethyl | Tetrahydrofuran-3-yl | H |
| 94 | " | Allyl | " | H |
| 95 | Ethyl | Ethyl | " | H |
| 96 | " | Allyl | " | H |
| 97 | Propyl | Ethyl | (6-H)-4,5-Dihydropyran-3-yl | H |
| 98 | " | Allyl | " | H |
| 101 | Ethyl | " | (2-H)-2,6-Dimethyl-5,6-dihydropyran-3-yl | H |
| 102 | " | Ethyl | " | H |
| 103 | Propyl | " | 2,6-Dimethyltetrahydropyran-3-yl | H |
| 104 | " | Allyl | " | H |
| 105 | Ethyl | Ethyl | " | H |
| 106 | " | Allyl | " | H |

| Verbindung Nr. | $R^1$ | $R^2$ | $X_n-Y$ | Z |
|---|---|---|---|---|
| 107 | Propyl | Ethyl | 1,3-Dioxep-5-yl | H |
| 108 | " | Allyl | " | H |
| 109 | " | Ethyl | 2-(1,3-Dithian-2-yl-)ethyl | H |
| 110 | " | Allyl | " | H |
| 111 | " | 3-Chlorallyl | " | H |
| 113 | Calciumsalz der Verbindung Nr. 26 | | | |
| 114 | Kupfersalz der Verbindung Nr. 26 | | | |
| 115 | Natriumsalz der Verbindung Nr. 79 | | | |
| 116 | Natriumsalz der Verbindung Nr. 19 | | | |
| 117 | Calciumsalz der Verbindung Nr. 19 | | | |

Die an diesen Verbindungen festgestellten 1H-NMR-spektroskopischen Daten sind in folgender Tabelle aufgeführt. Die chemischen Verschiebungen wurden auf Tetramethylsilan als internen Standard bezogen und in δ-Werten (ppm) angegeben.

Als Lösungsmittel diente $CDCl_3$; Abkürzungen für die Signalstrukturen

| | |
|---|---|
| s | Singulett |
| d | Dublett |
| t | Triplett |
| q | Quartett |
| m | Multiplett mit mehr als vier Linien |

| Verbindung Nr. | Charakteristische Signale | | |
|---|---|---|---|
| | 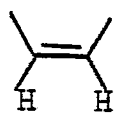 | $O-CH_2$ | $COOCH_3$ |
| 1 | - | 4,09 (q) | 3,75 (s) |
| 2 | - | 4,51 (d) | - |
| 3 | - | 4,51 (d) | 3,77 (s) |
| 4 | - | 4,51 (d) | 3,78 (s) |
| 5 | - | 4,11 (q) | - |
| 6 | - | 4,52 (d) | - |
| 7 | - | 4,51 (d) | 3,76 (s) |
| 8 | - | 4,08 (q) | - |
| 9 | - | 4,50 (d) | 3,78 (s) |
| 10 | - | 4,08 (q) | - |
| 11 | - | 4,58 (d) | - |
| 12 | - | 4,09 (q) | 3,74 (s) |
| 13 | - | 4,54 (d) | 3,78 (s) |
| 14 | - | | |
| 15 | - | 4,06 (q) | 3,69 (s) |
| 16 | - | 4,51 (d) | 3,70 (s) |
| 17 | 5,75 (s) | | 3,78 (s) |

| Verbindung Nr. | Charakteristische Signale | | |
|---|---|---|---|
| | 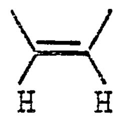 | $O-CH_2$ | $COOCH_3$ |
| 18 | 5,75 (s) | 4,50 (d) | 3,75 (s) |
| 19 | 5,60 (s) | 4,10 (q) | |
| 20 | 4,65 (m)<br>6,20 (m) | 4,10 (q) | 3,75 (s)+) |
| 21 | 4,70 (m)<br>6,30 (m) | 4,60 (d) | 3,70 (s) |
| 22 | - | 4,11 (q) | 3,75 (s)<br>3,80 (s) |
| 23 | - | 4,52 (d) | 3,75 (s)<br>3,80 (s) |
| 24 | - | 4,12 (q) | - |
| 25 | - | 4,51 (d) | - |
| 26 | - | 4,05 (q) | - |
| 31 | - | 4,50 (m) | - |
| 32 | - | 4,56 (s) | - |
| 44 | - | 4,50 | - |
| 68 | - | 4,89 (s) | - |

+) Die Aufspaltung der Estersignale wird durch Diasteromerie hervorgerufen.

Die Anwendung als Herbizid erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Streichen, Tränken, Vernebeln, Verstäuben, Verstreuen oder Gießen. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen

von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoran usw., stark polare Lösungsmittel, z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung vom Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Herbizide enthalten z.B. 5 bis 95 % (Gew.-%) insbesondere 10 bis 80 % Wirkstoff.

An oberflächenaktiven Stoffen sind zu nennen:
Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphtha-

BAD ORIGINAL

linderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes Isooctylphenol-, Octylphenol-, Nonylphenol, Alkylphenolpolyglykoläther. Tributylphenylpolyglykolether, Alkylarylpolyätheralkoholate, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

<u>Beispiel a</u>

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel b

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-mono-äthanolamid, 2 Gewichtsteilen Calciumsalz der Dodecylbenzolsulfonsäure und 2 Gewichtsteilen des Anlagerungsproduktes von 40 Mol Äthylenoxid an 1 Mol Ricinusöl besteht.

Beispiel c

20 Gewichtsteile der Verbindung 3 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Äthylenoxid an 1 Mol Isooctylphenol und 5 Gewichtsteilen des Anlagerungsproduktes von 40 Mol Äthylenoxid an 1 Mol Ricinusöl besteht.

Beispiel d

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht.

Beispiel e

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel f

5 Gewichtsteile der Verbindung 1 werden mit 95 Gewichtsteilen feinteiligem Kaolin innig vermischt. Man erhält auf diese Weise ein Stäubemittel, das 5 Gewichtsprozent des Wirkstoffs enthält.

Beispiel g

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion.

Beispiel i

20 Teile des Wirkstoffs 1 werden mit 12 Teilen Calciumsalz der Dodecylbenzolsulfonsäure, 8 Teile Fettalkohol-polyglykoläther, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Wirkung der neuen Cyclohexan-1,3-dionderivate auf das Wachstum von Pflanzen aus der Gräserfamilie (Gramineen) und breitblättrigen Kulturpflanzen läßt sich durch Gewächshaus- und Freilandversuche zeigen. Dabei können auch Kulturpflanzen aus der Familie der Gramineen absterben oder stark geschädigt werden. Dies kann in der Praxis durchaus erwünscht sein, da auch Kulturpflanzen zu unerwünschten Pflanzen werden können, wenn sie aus im Boden zurückgebliebenem Samen in einer anderen Kultur aufwachsen, wie z.B. Ausfallgerste (voluntary barley) in Winterraps oder Soghum in Sojabohnenfeldern.

Als Kulturgefäße für die Versuche dienten Plastikblumentöpfe mit 300 $cm^3$ Inhalt und lehmigem Sand mit etwa 1,5 % Humus als Substrat. Bei Soja wurde etwas Torf (peat) zugemischt, um ein besseres Wachstum zu gewährleisten. Die Samen der Testpflanzen wurden nach Arten getrennt flach eingesät.

Bei der Vorauflaufbehandlung wurden die Wirkstoffe auf die Erdoberfläche aufgebracht. Sie wurden hierzu in Wasser als Verteilungsmittel suspendiert oder emulgiert und mittels fein verteilender Düsen gespritzt. Bei dieser Applikationsmethode betrug die Aufwandmenge 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Die Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Pflanzen je nach Wuchsform bis zu einer Wuchshöhe von 3 bis 15 cm

an. Die Aufwandmengen für die Nachauflaufbehandlung variierte je nach Wirkstoff und Einsatzziel. Sie betrugen 0,125, 0,25, 0,5 und 1,0 kg Wirkstoff/ha.

Als Vergleichsbeispiel (DE-AS 24 39 104) dienten jeweils im Nachauflaufverfahren

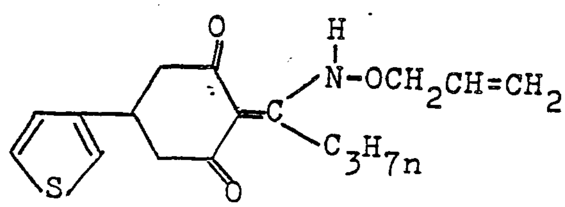

A

(DE-AS 2 439 104)

mit 0,25 kg/ha
sowie

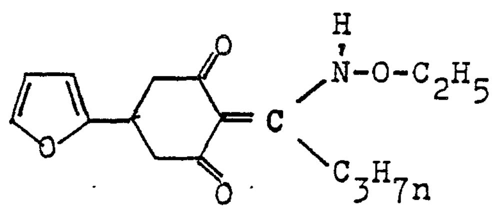

B

und

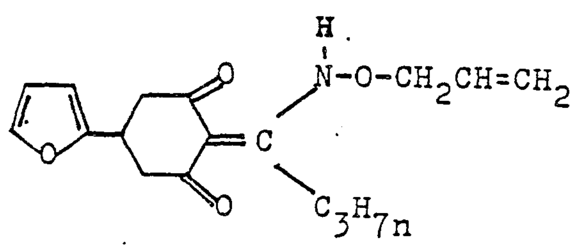

C

mit je 0,25 und 0,5 kg/ha.

Bei der Durchführung der Gewächshausversuche hielt man wärmeliebende Arten in wärmeren Bereichen (20 bis 35°C) und solche gemäßigter Klimate bevorzugt bei 10 bis 20°C. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wird nach einer Skala von 0 bis 100. Dabei bedeutet 0 keine Schädigung oder normaler Aufgang und 100 kein Aufgang bzw. völlige Zerstörung zumindest der oberirdischen Sproßteile.

Bei den ergänzend herangezogenen Feldversuchen wurden die Mittel auf Kleinparzellen ebenfalls in Wasser als Verteilungsmittel emulgiert oder suspendiert im Nachauflaufverfahren ausgebracht. Man benutzte hierzu eine auf einen Traktor montierte Parzellenspritze. Die Aufwandmengen betrugen 0,25 kg Wirkstoff/ha. Das Bekämpfungsziel war Ausfallgerste (voluntary barley) in jungem Winterraps.

Für die Darstellung der Ergebnisse wurden folgende Testpflanzen herangezogen:

| Botanischer Name | Deutscher Name | Englischer Name |
|---|---|---|
| Alopecurus myosuroides | Ackerfuchsschwanz | blackgrass |
| Avena fatua | Flughafer | wild oats |
| Avena sativa | Hafer | oats |
| Beta vulgaris | Zuckerrüben | suggarbeets |
| Brassica napus | Raps | rape seed |
| Bromus tectorum | Dach-Trespe | downy brome |
| Echinochloa crus-galli | Hühnerhirse | barnyardgrass |
| Gossypium hirsutum | Baumwolle | cotton |
| Glycine max. | Sojabohnen | soybeans |
| Hordeum vulgare | Gerste | barley |
| Lolium multiflorum | Ital. Raygras | annual ryegrass |
| Rottboellia exaltata | - | itchgrass |
| Setaria spp. | Borstenhirsearten | foxtail |
| Sorghum bicolor | Mohrenhirse | sorghum |
| Sorghum halepense | Sudangras | Johnsongrass |
| Triticum aestivum | Weizen | wheat |
| Zea mays | Mais | indian corn |

Die Ergebnisse zeigen, daß die neuen Verbindungen bei Nachauflaufanwendung zur Bekämpfung von unerwünschten Pflanzen aus der Familie der Gräser (Gramineen) geeignet sind. Dabei kann es sich um typische Ungrasarten handeln, wie z.B. Flughafer (Avena fatua) oder um Kulturpflanzen aus der Familie der Gramineen, welche am falschen Standort wachsend zu unerwünschten Pflanzen werden (z.B. Mais in einem Sojabohnenfeld). Einzelne Verbindungen, bekämpfen einerseits unerwünschte Gräser, andererseits weisen sie neben ihrer guten Selektivität für breitblättrige Kulturen gleichzeitig ein hohes Maß an Verträglichkeit für Weizen, der botanisch wiederum zur Gräserfamilie gehört, auf.

Die Prüfung der herbiziden Wirkung bei Nachauflaufanwendung von 0,25 kg Wirkstoff/ha der Verbindung Nr. 26 erbrachte gegen neun Beispielsgrasarten einen durchschnittlichen Bekämpfungswert von 81. Bei derselben Aufwandmenge und der gleichen Anwendungsmethode erreichte die Verbindung Nr. 24 einen Wert von 74.

Das bekannte Vergleichsmittel A hatte dagegen ebenfalls bei Nachauflaufanwendung von 0,25 kg Wirkstoff/ha gegen dieselben Grasarten nur eine durchschnittliche Wirkung von 52 %. Auch die beiden weiteren Vergleichsmittel B und C zeigten eine vergleichsweise nur schwache herbizide Aktivität.

Breitblättrige Kulturpflanzen, wie Baumwolle (Gossypium hirsutum), Soja (Glycine max.), Zuckerrüben (beta vulgaris) und Raps (Brassica napus) blieben bei diesen Behandlungen völlig ohne Schädigung oder zeigten nur ganz unwesentliche Beeinträchtigungen des Wuchses. Daraus resultiert für die neuen Verbindungen ein hohes Maß an Selektivität für dikotyle Kulturen. Darüber hinaus bekämpften einzelne der neuen Verbindungen, wie z.B. die Nr. 31 und

1, mit 0,25 kg Wirkstoff/ha unerwünschte Gräser, wie Ackerfuchsschwanz und Hirsen und verhielten sich dabei gleichzeitig selektiv für das Nutzgras Weizen.

Was die herbizide Aktivität betrifft, so konnten in einer Reihe weiterer Beispiele die Wirkung der neuen Verbindungen gegen Pflanzenarten aus der Gräserfamilie nachweisen, z.B. die Nr. 2, 10, 11, 19, 24 und 26.

In den beschriebenen Gewächshausversuchen erbrachten ferner bei Nachauflaufanwendung die Verbindungen Nr. 1, 4, 5, 8, 31, 32, 36 und 37 einen vergleichsweise guten Bekämpfungserfolg.

In Freilandversuchen wurde bei Nachauflaufanwendung von 0,25 kg Wirkstoff/ha der Verbindungen Nr. 10, 11 und 26 Ausfallgerste in Raps selektiv bekämpft.

Neben den Nachauflaufwirkungen wurden auch positive Ergebnisse bei Vorauflaufanwendung der neuen Verbindungen im Gewächshaus erzielt. So wirkten bei 3,0 kg Wirkstoff/ha bei dieser Anwendungsmethode die Verbindungen Nr. 2, 5, 8, 10, 14, 19, 26, 32, 36, 37, 48, 49, 54, 55, 77 und 78 stark herbizid gegen die grasartigen Beispielspflanzen Hafer, Weidelgras und Hühnerhirse. Ebenso hatten die Verbindungen Nr. 1, 3 und 4 bei Vorauflaufanwendung von 3,0 kg Wirkstoff/ha im Gewächshaus eine beachtliche herbizide Aktivität gegen diese eben genannten Grasarten.

In Anbetracht der guten Verträglichkeit können die neuen Herbizide oder diese enthaltende Mittel noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

Beispielsweise kommen folgende Kulturpflanzen in Betracht.

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |

| Botanischer Name | Deutscher Name | Englischer Name |
|---|---|---|
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |

| Botanischer Name | Deutscher Name | Englischer Name |
|---|---|---|
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

| Botanischer Name | Deutscher Name | Englischer Name |
|---|---|---|
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Sasamum indicum | Sesam | Sesame |
| Solanum tuberosum | Kartoffel | Irish potatoes |
| Sorghum bicolor (s. vulgare) | Mohrenhirse (Unterblattspritzung) (post-directed) | sorghum |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais (Unterblattspritzung) (post-directed) | Indian corn, sweet corn maize |

Zur Verbreiterung des Wirkungsspektrums und zur Erzielung auch synergistischer Effekte können die neuen Cyclohexan-1,3-dion-Derivate mit bekannten Cyclohexan-1,3-dion-Derivaten und mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Frage. Sinnvolle Mischungen ergeben die erfindungsgemäßen Verbindungen je nach Einsatzziel mit folgenden Wirkstoffen:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl)-3(2H)-pyridazinon
5-Methylamino-4-chlor-2-(3-alpha-alpha-beta-beta-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon
3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid

1-Cyan-8-methyl-3-(1-methylethyl)-2,1,3-benzothiadiazin--4(3H)-on-2,2-dioxid

1-Cyan-3-(1-methylethyl)-2,1,3-benzothiadiazin-4(3H)-on--2,2-dioxid

1-Azidomethyl-3-(1-methylethyl)-2,1,3-benzothiadiazin--4(3H)-on-2,2-dioxid

3-(1-methylethyl)-1H-(pyridino-[3,2-e]2,1,3-thiadiazin--(4)-on-2,2-dioxid

N-(1-Ethylpropyl)-2,6-dinitro-3,4-dimethylanilin

N-(1-Methylethyl)-N-ethyl-2,6-dinitro-4-trifluormethyl--anilin

N-n-Propyl-N-beta-chlorethyl-2,6-dinitro-4-trifluormethyl--anilin

N-n-Propyl-N-cyclopropylmethyl-2,6-dinitro-4-trifluor--methyl-anilin

N-Bis(n-propyl)-2,6-dinitro-3-amino-4-trifluormethylanilin

N-Bis(n-propyl)-2,6-dinitro-4-methyl-anilin

N-Bis(n-propyl)-2,6-dinitro-4-methylsulfonyl-anilin

N-bis(n-propyl)-2,6-dinitro-4-aminosulfonyl-anilin

Bis(ß-chlorethyl)-2,6-dinitro-4-methyl-anilin

N-Ethyl-N-(2-methylallyl)-2,6-dinitro-4-trifluormethyl--anilin

N-Methylcarbaminsäure-3,4-dichlorbenzylester

N-Methylcarbaminsäure-2,6-di(tert.butyl)-4-methylphenyl--ester

N-Phenylcarbaminsäure-isopropylester

N-3-Fluorphenylcarbaminsäure-3-methoxypropyl-2-ester

N-3-Chlorphenylcarbaminsäure-isopropylester

N-3-Chlorphenylcarbaminsäure-butin-1-yl-3-ester

N-3-Chlorphenylcarbaminsäure-4-chlor-butin-2-yl-1-ester

N-3,4-Dichlorphenylcarbaminsäure-methylester

N-(4-Amino-benzolsulfonyl)-carbaminsäure-methylester

O-(N-Phenylcarbamoyl)-propanonoxim
N-Ethyl-2-(phenylcarbamoyl)-oxypropionsäureamid
3'-N-Isopropyl-carbamoyloxy-propionsäureanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-N'-3-chlor-4-fluorphenylcarbamoyloxy)-phenyl)-carbamat
Ethyl-N-(3-N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester

N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat

N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl,alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)

2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephtalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')-]-ethan (Salze)
1-(4-Chlorphenoxy-3,3-dimethyl-1(1H-1,2,4-triazol-1-yl)-2-butanon
N,N-Diallylchloracetamid

N-Isopropyl-2-chloracetanilid
N-(1-Methyl-propin-2-yl)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chlor--acetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetatanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy-N-methoxy-acetamid
2-(Alpha-Naphtoxy)-N,N-diethylpropionamid
2,2-Diphenyl-N,N-dimethylacetamid
Alpha(3,4,5-Tribrompyrazol-1-yl)-N,N-dimethylpropionamid
N-(1,1-Dimethylpropinyl)-3,5-dichlorbenzamid

N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethyl-anilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenamid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion

2-(3-tert.Butylcarbamoyl-oxyphenyl)-4-methyl-1,2,4-oxadiazolidin-3,5-dion

2-(3-iso-Propylcarbamoyl-oxyphenyl)-4-methyl-1,2,4-oxadiazolidin-3,5-dion

2-Phenyl-3,1-benzoxazinon-(4)

(4-Bromphenyl)-3,4,5,9,10-pentaazatetracyclo-[5,4,1,$0^{2,6}$,0, $^{8,11}$]-dodeca-3,9-dien

2-Ethoxy-2,3-dihydro-3,3-dimethyl-5-benzofuranyl-methan-sulfonat

2-Ethoxy-2,3-dihydro-3,3-dimethyl-5-benzofuranyl-dimethyl-aminosulfonat

2-Ethoxy-2,3-dihydro-3,3-dimethyl-5-benzofuranyl-(N-methyl-N-acetyl)-aminosulfonat

3,4-Dichlor-1,2-benzisothiazol

N-4-Chlorphenyl-allylbernsteinsäureimid

2-Methyl-4,6-dinitrophenol (Salze, Ester)

2-sec.Butyl-4,6-dinitrophenol (Salze,)

2-sec.Butyl-4,6-dinitrophenol-acetat

2-tert.Butyl-4,6-dinitrophenol-acetat

2-tert.Butyl-4,6-dinitrophenol (Salze)

2-tert.Butyl-5-methyl-4,6-dinitrophenol (Salze)

2-tert.Butyl-5-methyl-4,6-dinitrophenol-acetat

2-sec.Amyl-4,6-dinitrophenol (Salze, Ester)

1-(Alpha,alpha-Dimethylbenzyl)-3-(4-methylphenyl)-harnstoff

1-Phenyl-3-(2-methylcyclohexyl)-harnstoff

1-Phenyl-1-benzoyl-3,3-dimethyl-harnstoff

1-(4-chlorphenyl)-1-benzoyl-3,3-dimethyl-harnstoff

1-(4-chlorphenyl)-3,3-dimethyl-harnstoff

1-(4-Chlorphenyl)-3-methyl-3-butin-1-yl-3-harnstoff

1-(3,4-Dichlorphenyl)-3,3-dimethyl-harnstoff

1-(3,4-Dichlorphenyl)-1-benzoyl-3,3-dimethyl-harnstoff

1-(3,4-Dichlorphenyl)-3-methyl-3-n-butyl-harnstoff

1-(4-i-Propylphenyl)-3,3-dimethyl-harnstoff

1-(3-Trifluormethylphenyl)-3,3-dimethyl-harnstoff

1-(3-Alpha,alpha,beta,beta-Tetrafluorethoxyphenyl)--3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]--3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl--harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid

3-Phenyl-4-hydroxy-6-chlorpyridazin
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden] -6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

Alpha-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy-acrylat

9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol

N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitrophenylether
2-Chlor-4-trifluormethylphenyl-3-(ethoxycarbonyl)-methylthio-4-nitrophenylether
2,4,6-Trichlorphenyl-3-(ethoxycarbonyl)-methylthio-4-nitrophenylether
2-[1-(N-Ethoxamino)-butyliden]-5-(2-ethylthiopropyl)-3--hydroxy-cyclohexen-(2)-on-(1) (Salze)
2-[1-(N-Ethoxamino)-butyliden]-5-(2-phenylthiopropyl)-3--hydroxy-cyclohexen-(2)-on-(1) (Salze)

4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha-alpha-beta-trifluor-beta-bromethoxyphenyl)-3-(2H)-pyridazinon
2,4-Dichlorphenyl-3'-ethoxy-ethoxy-ethoxy-4'-nitrophenyl--ether
2,3-Dihydro-3,3-dimethyl-5-benzofuranyl-ethansulfonat
N-(4-Methoxy-6-methyl-1,3,5-triazin-2-yl-aminocarbonyl)-2--chlorbenzolsulfonamid
1-(3-Chlor-4-ethoxyphenyl)-3,3-dimethylharnstoff
2-Methyl-4-Chlorphenoxy-thioessigsäureethylester
2-Chlor-3,5-dijod-4-acetoxy-pyridin

1-{4-[2-(4-Methylphenyl)-ethoxy]-phenyl}-3-methyl-3-methoxyharnstoff

2,6-Dimethyl-N-(pyrazol-1-yl-methylenoxymethyl)-2-chloracetanilid

2-Methyl-6-ethyl-N-(pyrazol-1-yl-methylenoxymethyl)-2-chloracetanilid

1-(Alpha-2,4-Dichlorphenoxypropionsäure)-3-(O-methylcarbamoyl)-anilid

1-(Alpha-2-Brom-4-chlorphenoxypropionsäure)-3-(O-methylcarbamoyl)-anilid

2-Methyl-6-ethyl-N-(pyrazol-1-yl-ethylenoxymethyl)-2-chloracetanilid

Methyl-N-dichlorfluormethylsulfenyl-[3-(N'-dichlorfluormethylsulfenyl-N'-phenylcarbamoyl-oxy)-phenyl]-carbamat

Methyl-N-dichlorfluormethylsulfenyl-[3-(N'-dichlorfluormethylsulfenyl-N'-3-methylphenylcarbamoyl-oxy)-phenyl]-carbamat

N-(Pyrazol-1-yl-methyl)-pyrazol-1-yl-essigsäure-2,6-dimethylanilid

N-(Pyrazol-1-yl-methyl)-1,2,4-triazol-1-yl-essigsäure-2,6-dimethylanilid

2-(3-Trifluormethylphenyl)-4H-3,1-benzoxazin-4-on

2-(2-Thienyl)-4H-3,1-benzoxazin-4-on

2-(3-Pentafluorethoxyphenyl)-4H-3,1-benzoxazin-4-on

2-(3-Trifluormethylthio-phenyl)-4H-3,1-benzoxazin-4-on

2-(3-Difluor-chlormethoxyphenyl)-4H-3,1-benzoxazin-4-on

5-Nitro-2-(3-trifluormethyl-phenyl)-4H-3,1-benzoxazin-4-on

5-Chlor-2-(3-trifluormethoxyphenyl)-4H-3,1-benzoxazin-4-on

5-Chlor-2-[(3-alpha-alpha-beta-beta)-tetrafluorethoxyphenyl]-4H-3,1-benzoxazin-4-on

5-Fluor-2-[(3-alpha-alpha-beta-beta)-tetrafluorethoxyphenyl]-4H-3,1-benzoxazin-4-on

5-Chlor-2-(4-Difluorchlormethoxyphenyl)-4H-3,1-benzoxazin-4-on

5-Fluor-2-(4-difluorchlormethoxyphenyl)-4H-3,1-benzoxazin-4-on
5-Fluor-2-(phenyl)-4H-3,1-benzoxazin-4-on
5-Fluor-2-(3-difluormethoxyphenyl)-4H-3,1-benzoxazin-4-on
5-Chlor-2-(phenyl)-4H-3,1-benzoxazin-4-on
3-(3,5-Dichlorphenyl)-4-methoxycarbonyl-5-methylpyrazol
3-(3-Chlorphenyl)-4-methoxycarbonyl-5-methylpyrazol
3-(3-Fluorphenyl)-4-methoxycarbonyl-5-methylpyrazol
1-Acetyl-3-(3-fluorphenyl)-4-methoxycarbonyl-5-methylpyrazol
1-Acetyl-3-(3-chlorphenyl)-4-methoxycarbonyl-5-methylpyrazol
1-Acetyl-3-(3-bromphenyl)-4-methoxycarbonyl-5-methylpyrazol
1-Acetyl-3-(3,5-dichlorphenyl)-4-methoxycarbonyl-5-methylpyrazol
1-Acetyl-3-thienyl-4-methoxycarbonyl-5-methylpyrazol
N-3-Chlor-4-isopropylphenyl-thiolcarbaminsäuremethylester
N-3-Methyl-4-fluorphenyl-thiolcarbaminsäuremethylester
N-3-Chlor-4-isopentylphenyl-thiolcarbaminsäuremethylester
N-3-Chlor-4-difluormethoxyphenyl-thiolcarbaminsäuremethylester
N-3-Chlor-4-(1-chlorisopropyl)-phenyl-thiolcarbaminsäuremethylester

1-(2-Fluorphenyl)-3-methyl-5-iminoimidazolidin-2-on
1-(3-Isopropylphenyl)-3-methyl-5-iminoimidazolidin-2-on
1-(4-Isopropylphenyl)-3-methyl-5-iminoimidazolidin-2-on
1-[3-(1,1,2,2-Tetrafluorethoxy)-phenyl]-3-methyl-5-iminoimidazolidin-2-on
1-(3,4-Dichlorphenyl)-3-methyl-5-iminoimidazolidin-2-on
1-(3,4-Difluorphenyl)-3-methyl-5-iminoimidazolidin-2-on

6-Methyl-3-methoxy-5,6-dihydro-1,2,4,6-thiatriazin-5-on-1,1-dioxid

6-Methyl-3-methoxy-5,6-dihydro-1,2,4,6-thiatriazin-5-on-1,1-dioxid Natriumsalz

6-n-Propyl-3-methoxy-5,6-dihydro-1,2,4,6-thiatriazin-5-on-1,1-dioxid

6-Methyl-3-ethoxy-5,6-dihydro-1,2,4,6-thiatriazin-5-on-1,1-dioxid

6-n-Propyl-3-ethoxy-5,6-dihydro-1,2,4,6-thiatriazin-5-on-1,1-dioxid Natriumsalz

6-Methyl-3-isopropoxy-5,6-dihydro-1,2,4,6-thiatriazin-5-on-1,1-dioxid

6-n-Propyl-3-isopropoxy-5,6-dihydro-1,2,4,6-thiatriazin-5-on-1,1-dioxid

6-Isopropyl-3-sek.butoxy-5,6-dihydro-1,2,4,6-thiatriazin-5-on-1,1-dioxid Natriumsalz

N-3'-(2"-Chlor-4"-trifluormethylphenoxy)-6'-nitrobenzoyl-anthranilsäure

N-3'-(2"-Chlor-4"-trifluormethylphenoxy)-6'-nitrobenzoyl-anthranilsäuremethylester

N-3'-(2"-Chlor-4"-trifluormethylphenoxy)-6'-nitrobenzoyl-anthranilsäure Natriumsalz

N-3'-(2"-Chlor-4"-trifluormethylphenoxy)-6'-nitrobenzoyl-3-chloranthranilsäure

N-3'-(2"-Chlor-4"-trifluormethylphenoxy)-benzoyl-3-chloranthranilsäure

N-3'-(2"-Chlor-4"-trifluormethylphenoxy)-benzoyl-3-methylanthranilsäure

N-3'-(2"-Chlor-4"-trifluormethylphenoxy)-benzoylanthranilsäure

N-3'-(2",4"-Dichlorphenoxy)-6'-nitrobenzoylanthranilsäure

N-[3'-(2"-Chlor-4"-trifluormethylphenoxy)-6'-nitrophenyl]-4H-1,3-benzoxazin-4-on

N-[3'-(2"-Chlor-4"-trifluormethylphenoxy)-6'-nitrophenyl]-4H-1,3-8-methoxybenzoxazin-4-on

5-Chlor-2-(3-trifluormethyl-phenyl)-4H-3,1-benzoxazin-4-on
5-Fluor-2-(3-trifluormethyl-phenyl)-4H-3,1-benzoxazin-4-on
5-Fluor-2-(3-difluor-chlormethyl-phenyl)-4H-3,1-benzoxazin--4-on
5-Chlor-2-(3-difluor-chlormethyl-phenyl)-4H-3,1-benzoxazin--4-on
1-[5-(3-Fluorbenzylthio)-thiadiazolyl-2]-1-methyl-3-methylharnstoff

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

## Patentansprüche

1. Cyclohexandionderivat der allgemeinen Formel

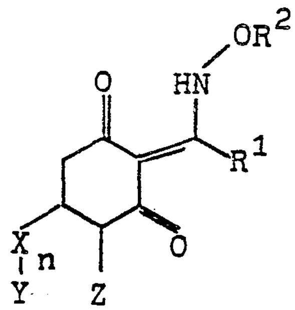

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

2. Herbizid, enthaltend ein Cyclohexandionderivat der allgemeinen Formel

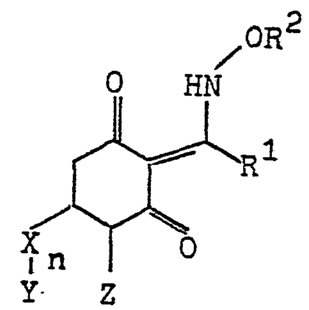

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

3. Herbizid, enthaltend einen festen oder flüssigen Trägerstoff und ein Cyclohexandionderivat der allgemeinen Formel

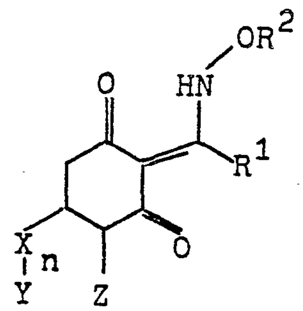

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

4. Verfahren zur Herstellung eines Herbizids, dadurch gekennzeichnet, daß man einen festen oder flüssigen Trägerstoff vermischt mit einem Cyclohexandionderivat der allgemeinen Formel

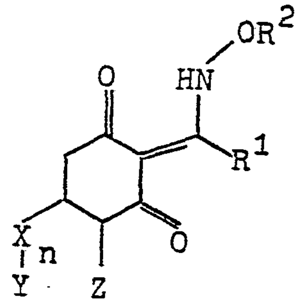

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

5. Verfahren zur Bekämpfung unerwünschten Pflanzenwuchses, dadurch gekennzeichnet, daß man den Boden oder die Pflanzen behandelt mit einem Cyclohexandionderivat der allgemeinen Formel

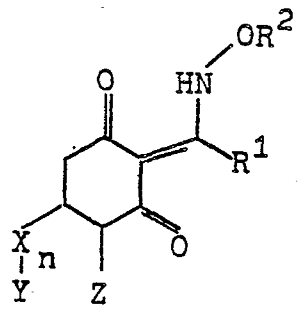

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

6. Verfahren zur Herstellung eines Cyclohexandionderivats der allgemeinen Formel

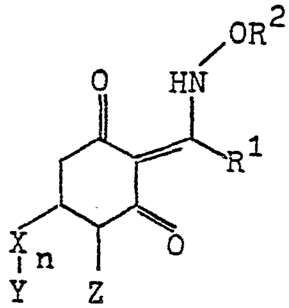

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung,

<u>dadurch gekennzeichnet,</u> daß man eine Verbindung der allgemeinen Formel

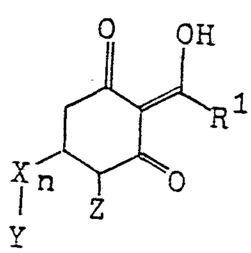

mit einer Ammoniumverbindung der Formel $R^2-O-NH_3^+ A^-$, in denen $R^1$, $R^2$, X, Y, Z die in Anspruch 1 genannten Bedeutungen haben und $A^-$ ein Anion bedeutet, in einem inerten Lösungsmittel bei einem pH-Bereich von 2 bis 7 und bei Temperaturen zwischen 0 und 80°C umsetzt.

7. Cyclohexandionderivat, ausgewählt aus der Gruppe, bestehend aus
2-(1-Ethyloxiaminobutyliden)-5-[3-(4-methyltetrahydropyranyl)]-cyclohexan-1,3-dion,
2-(1-Allyloxiaminobutyliden)-5-[3-(4-methyltetrahydropyranyl)]-cyclohexan-1,3-dion,
2-(1-Ethyloxiaminobutyliden)-5-[3-(2-H)-5,6-dihydropyranyl]-cyclohexan-1,3-dion.

8. Herbizid, enthaltend ein Cyclohexandionderivat, ausgewählt aus der Gruppe, bestehend aus
2-(1-Ethyloxiaminobutyliden)-5-[3-(4-methyltetrahydropyranyl)]-cyclohexan-1,3-dion,
2-(1-Allyloxiaminobutyliden)-5-[3-(4-methyltetrahydropyranyl)]-cyclohexan-1,3-dion,
2-(1-Ethyloxiaminobutyliden)-5-[3-(2-H)-5,6-dihydropyranyl]-cyclohexan-1,3-dion.

9. Herbizid, enthaltend einen festen oder flüssigen Trägerstoff und ein Cyclohexandionderivat, ausgewählt aus der Gruppe, bestehend aus
2-(1-Ethyloxiaminobutyliden)-5-[3-(4-methyltetrahydropyranyl)]-cyclohexan-1,3-dion,
2-(1-Allyloxiaminobutyliden)-5-[3-(4-methyltetrahydropyranyl)]-cyclohexan-1,3-dion,
2-(1-Ethyloxiaminobutyliden)-5-[3-(2-H)-5,6-dihydropyranyl]-cyclohexan-1,3-dion.

Patentansprüche (für Österreich)

1. Herbizid, enthaltend ein Cyclohexandionderivat der allgemeinen Formel

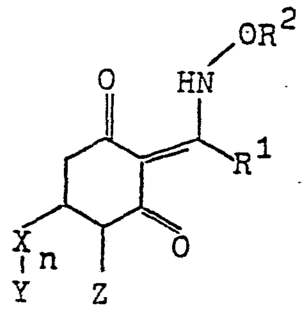

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

2. Herbizid, enthaltend einen festen oder flüssigen Trägerstoff und ein Cyclohexandionderivat der allgemeinen Formel

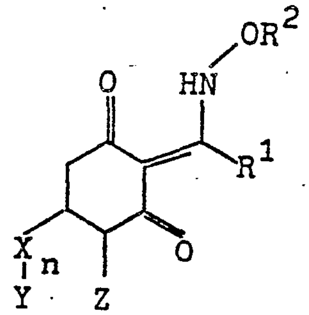

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

3. Verfahren zur Herstellung eines Herbizids, <u>dadurch gekennzeichnet</u>, daß man einen festen oder flüssigen Trägerstoff vermischt mit einem Cyclohexandionderivat der allgemeinen Formel

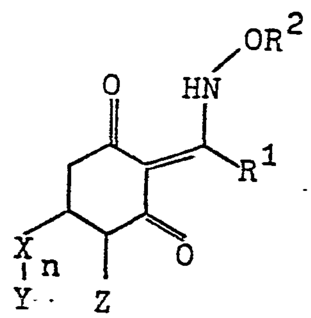

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

7. Verfahren zur Bekämpfung unerwünschten Pflanzenwuchses, dadurch gekennzeichnet, daß man den Boden oder die Pflanzen behandelt mit einem Cyclohexandionderivat der allgemeinen Formel

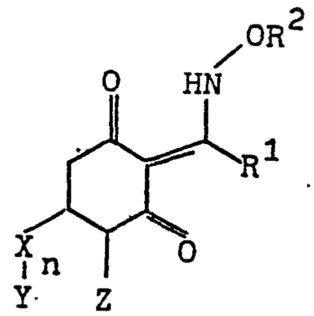

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung.

5. Verfahren zur Herstellung eines Cyclohexandionderivats der allgemeinen Formel

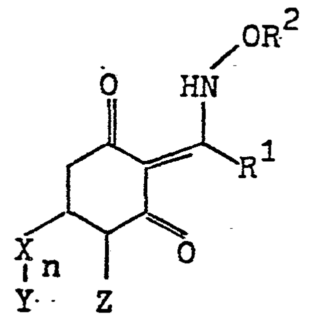

in der

$R^1$ Alkyl mit 1 bis 4 Kohlenstoffatomen

$R^2$ Alkyl mit 1 bis 4 Kohlenstoffatomen, Alkenyl mit 3 bis 4 Kohlenstoffatomen, Alkinyl mit 3 bis 4 Kohlenstoffatomen oder Halogenalkenyl mit 3 oder 4 Kohlenstoffatomen und 1 bis 3 Halogenatomen

X geradkettiger oder verzweigter Alkylenrest mit 1 bis 5 Kohlenstoffatomen, gegebenenfalls phenylsubstituiert

n = 0 oder 1

Y einen nichtaromatischen Heterocyclus mit 4 bis 7 Atomen und keiner oder einer Doppelbindung im heterocyclischen Ring, enthaltend 1 oder 2 Heteroatome aus der Gruppe Schwefel, Stickstoff, Sauerstoff in beliebiger Reihenfolge, wobei der Heterocyclus gegebenenfalls substituiert ist durch Alkyl

Z Wasserstoff oder Methoxycarbonyl bedeutet sowie die Salze dieser Verbindung,

dadurch gekennzeichnet, daß man eine Verbindung der allgemeinen Formel

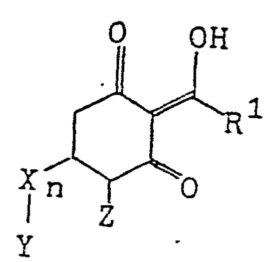

mit einer Ammoniumverbindung der Formel $R^2-O-NH_3^+ A^-$, in denen $R^1$, $R^2$, X, Y, Z die in Anspruch 1 genannten Bedeutungen haben und $A^-$ ein Anion bedeutet, in einem inerten Lösungsmittel bei einem pH-Bereich von 2 bis 7 und bei Temperaturen zwischen 0 und 80°C umsetzt.

6. Herbizid, enthaltend ein Cyclohexandionderivat, ausgewählt aus der Gruppe, bestehend aus
2-(1-Ethyloxiaminobutyliden)-5-[3-(4-methyltetrahydropyranyl)]-cyclohexan-1,3-dion,
2-(1-Allyloxiaminobutyliden)-5-[3-(4-methyltetrahydropyranyl)]-cyclohexan-1,3-dion,
2-(1-Ethyloxiaminobutyliden)-5-[3-(2-H)-5,6-dihydropyranyl]-cyclohexan-1,3-dion.

7. Herbizid, enthaltend einen festen oder flüssigen Trägerstoff und ein Cyclohexandionderivat, ausgewählt aus der Gruppe, bestehend aus
2-(1-Ethyloxiaminobutyliden)-5-[3-(4-methyltetrahydropyranyl)]-cyclohexan-1,3-dion,
2-(1-Allyloxiaminobutyliden)-5-[3-(4-methyltetrahydropyranyl)]-cyclohexan-1,3-dion,
2-(1-Ethyloxiaminobutyliden)-5-[3-(2-H)-5,6-dihydropyranyl]-cyclohexan-1,3-dion.

Europäisches Patentamt

# EUROPÄISCHER RECHERCHENBERICHT

0070370

Nummer der Anmeldung

EP 82 10 4389

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 439 104 (NIPPON SODA) *Seiten 1-6,21 nr. 118, Seiten 25-41* --- | 1-7 | C 07 D 309/06<br>C 07 D 309/22<br>C 07 D 307/14<br>C 07 D 307/16<br>C 07 D 317/28<br>C 07 D 317/30<br>C 07 D 319/06<br>C 07 D 335/02<br>C 07 D 339/06<br>A 01 N 43/02 |
| A | DE-A-2 524 577 (NIPPON SODA) *Seiten 1-11* ----- | 1-7 | |
| | | | RECHERCHIERTE SACHGEBIETE (Int. Cl. 3) |
| | | | C 07 D 309/00<br>C 07 D 317/00<br>C 07 D 319/00<br>C 07 D 339/00<br>C 07 D 307/00 |

Der vorliegende Recherchenbericht wurde für alle Patentansprüche erstellt.

| Recherchenort | Abschlußdatum der Recherche | Prüfer |
|---|---|---|
| DEN HAAG | 27-09-1982 | FRANCOIS J.C.L. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsatze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument